(19) Europäisches Patentamt
European Patent Office
Office européen des brevets

(11) Veröffentlichungsnummer : **0 024 747**
**B1**

(12) **EUROPÄISCHE PATENTSCHRIFT**

(45) Veröffentlichungstag der Patentschrift : **11.05.83**

(51) Int. Cl.[3] : **C 07 C131/00**// A01N37/22

(21) Anmeldenummer : **80106219.1**

(22) Anmeldetag : **22.10.79**

(60) Veröffentlichungsnummer der früheren Anmeldung nach Art. 76 EPÜ : **0010715**

(54) **Anilinomethyloxim-ether und Verfahren zu ihrer Herstellung.**

(30) Priorität : **03.11.78 DE 2847827**
**31.07.79 DE 2931103**

(43) Veröffentlichungstag der Anmeldung : **11.03.81 Patentblatt 81/10**

(45) Bekanntmachung des Hinweises auf die Patenterteilung : **11.05.83 Patentblatt 83/19**

(84) Benannte Vertragsstaaten : **BE CH DE FR GB IT NL**

(56) Entgegenhaltungen :
**DE A 2 702 119**
**GB A 1 488 462**

(73) Patentinhaber : **BAYER AG**
**Zentralbereich Patente, Marken und Lizenzen**
**D-5090 Leverkusen 1, Bayerwerk (DE)**

(72) Erfinder : **Stetter, Jörg, Dr.**
**Pahlkestrasse 3**
**D-5600 Wuppertal 1 (DE)**
Erfinder : **Draber, Wilfried, Dr.**
**In den Birken 81**
**D-5600 Wuppertal 1 (DE)**
Erfinder : **Regel, Erik, Ing.grad.**
**Bergerheide 72a**
**D-5600 Wuppertal 1 (DE)**
Erfinder : **Eue, Ludwig, Dr.**
**Paul-Klee-Strasse 36**
**D-5090 Leverkusen (DE)**
Erfinder : **Schmidt, Robert Rudolf, Dr.**
**Hahnenweg 5**
**D-5000 Köln 80 (DE)**

**EP 0 024 747 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Anilinomethyloxim-ether und Verfahren zu ihrer Herstellung

Die vorliegende Anmeldung betrifft neue Anilinomethyloxim-ether, mehrere Verfahren zu deren Herstellung sowie ihre Verwendung als Zwischenprodukte zur Herstellung von Oximethern, welche herbizide Eigenschaften besitzen.

Es ist bereits bekannt geworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977) sowie US-Patentschrift 3 442 945). Diese Verbindung ist jedoch nicht immer ausreichend wirksam und in ihrer Selektivität nicht immer ganz befriedigend.

Es wurden nun neue Anilinomethyloxim-ether der Formel

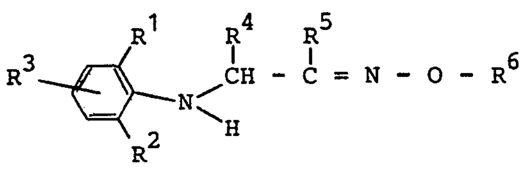

(I)

in welcher

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl oder gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen steht,

$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl, gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen oder gegebenenfalls im Arylteil durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aralkyl mit 6 bis 10 Kohlenstoffatomen im Arylteil und 1 bis 2 Kohlenstoffatomen im Alkylteil steht und außerdem

$R^5$ und $R^6$ gemeinsam für eine Alkylen-Gruppe stehen, gefunden.

Weiterhin wurde gefunden, daß man Anilinomethyloxim-ether der Formel (I) erhält, wenn man

a) Aniline der Formel

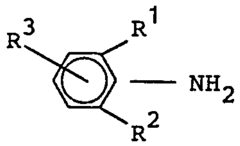

(II)

in welcher

$R^1$, $R^2$ und $R^3$ die oben angegebene Bedeutung haben,

α) mit substituierten Oxim-ethern der Formel

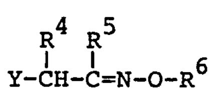

(III)

in welcher

$R^4$, $R^5$ und $R^6$ die oben angegebene Bedeutung haben und

Y für Halogen, den Mesylat- oder Tosylat-Rest steht, in Gegenwart eines Säurebinders und gegebenenfalls in Gegenwart eines Verdünnungsmittels umsetzt, oder

β) mit Propargylhalogeniden der Formel

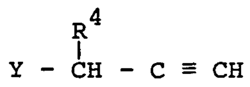

(IV)

in welcher

$R^4$ und Y die oben angegebene Bedeutung haben, in Gegenwart eines Säurebinders und gegebenenfalls in Gegenwart eines Verdünnungsmittels umsetzt, die entstehenden Propargyl-aniline der Formel

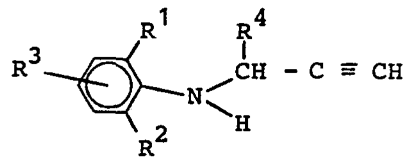

(V)

in welcher

$R^1$, $R^2$, $R^3$ und $R^4$ die oben angegebene Bedeutung haben, in üblicher Weise hydratisiert und die entstehenden Anilin-Derivate der Formel

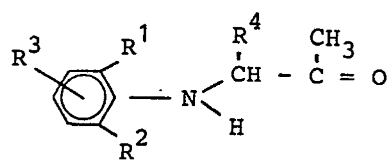

(VI)

in welcher

$R^1$, $R^2$, $R^3$ und $R^4$ die oben angegebene Bedeutung haben, mit Salzen von Hydroxylamin-(Derivaten) der Formel

$$H_2N—O—R^6 \qquad (VII)$$

in welcher

$R^6$ die oben angegebene Bedeutung hat, in Gegenwart eines Verdünnungsmittels und in Gegenwart eines Säurebindemittels umsetzt, oder

γ) mit Keto-Derivaten der Formel

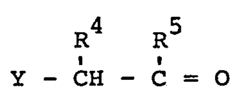

(VIII)

in welcher

$R^4$, $R^5$ und Y die oben angegebene Bedeutung haben, in Gegenwart eines Säurebinders und gegebenenfalls in Gegenwart eines Verdünnungsmittels umsetzt, und die entstehenden substituierten Anilin-Derivate der Formel

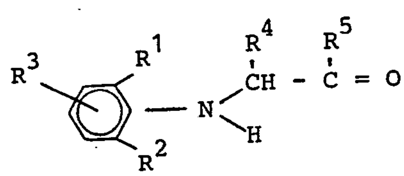

(IX)

in welcher

$R^1$, $R^2$, $R^3$, $R^4$ und $R^5$ die oben angegebene Bedeutung haben, mit Salzen von Hydroxylamin-(Derivaten) der Formel

$$H_2N—O—R^6 \qquad (VII)$$

in welcher

$R^6$ die oben angegebene Bedeutung hat, in Gegenwart eines Verdünnungsmittels und in Gegenwart

eines Säurebindemittels umsetzt, und daß man diejenigen Anilinomethyloxim-ether der Formel (I) erhält, in denen $R^6$ nicht für Wasserstoff steht, wenn man

b) Anilin-Derivate der Formel

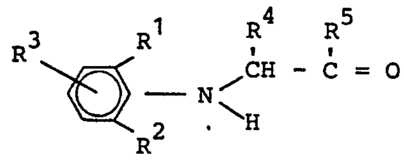

(IX)

in welcher

$R^1$, $R^2$, $R^3$, $R^4$ und $R^5$ die oben angegebene Bedeutung haben, mit Salzen des Hydroxylamins in Gegenwart eines Verdünnungsmittels und in Gegenwart eines Säurebindemittels umsetzt und die dabei entstehenden Oxime der Formel

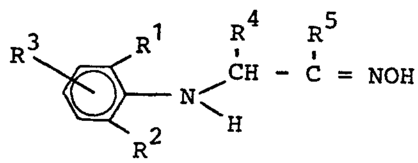

(X)

in welcher

$R^1$, $R^2$, $R^3$, $R^4$ und $R^5$ die oben angegebene Bedeutung haben, in Form ihrer Alkali-Salze mit Dimethylsulfat oder mit Halogeniden der Formel

$$X—R^7 \qquad (XI)$$

in welcher

$R^7$ für Alkyl, Alkenyl, Alkinyl, Alkoxyalkyl, Alkylthioalkyl, Dialkylaminoalkyl, Halogenalkyl, Alkoxycarbonylalkyl, gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen oder gegebenenfalls im Arylteil durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aralkyl mit 6 bis 10 Kohlenstoffatomen im Arylteil und 1 bis 2 Kohlenstoffatomen im Alkylteil steht und

X für Chlor oder Brom steht, in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der Formel (X) in situ erzeugt werden.

Die erfindungsgemäßen Anilinomethyloximether der Formel (I) eignen sich als Zwischenprodukte zur Synthese von Oximethern, welche herbizide Eigenschaften besitzen.

Überraschenderweise zeigen die Oximether, die sich aus den erfindungsgemäßen Anilinomethyloxim-ethern der Formel (I) durch Umsetzung mit Halogenessigsäurechloriden oder -bromiden bzw. -anhydriden herstellen lassen, bei sehr guter Unkrautwirkung insbesondere bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist. Die erfindungsgemäßen Stoffe stellen somit als Zwischenprodukte zur Synthese hochwertiger Herbizide eine wesentliche Bereicherung der Technik dar.

Die erfindungsgemäßen Anilinomethyloxim-ether sind durch die Formel (I) allgemein definiert. In der Formel (I) steht $R^1$ vorzugsweise für Wasserstoff sowie geradkettiges oder verzweigtes Alkyl und Alkoxy mit jeweils 1 bis 4 Kohlenstoffatomen. $R^2$ und $R^3$ sind gleich oder verschieden und stehen vorzugsweise für Wasserstoff, geradkettiges oder verzweigtes Alkyl und Alkoxy mit jeweils 1 bis 4 Kohlenstoffatomen, sowie für die Halogene Fluor, Chlor und Brom. $R^4$ steht vorzugsweise für Wasserstoff und geradkettiges oder verzweigtes Alkyl mit 1 bis 4 Kohlenstoffatomen. $R^5$ steht vorzugsweise für Wasserstoff, geradkettiges oder verzweigtes Alkyl mit 1 bis 4 Kohlenstoffatomen sowie für die übrigen Reste, die für den Substituenten $R^5$ bereits oben genannt wurden.

$R^6$ steht vorzugsweise für Wasserstoff, geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils

1 bis 4 Kohlenstoffatomen im Alkylteil und 1 bis 4 Kohlenstoffatomen im Alkoxy- bzw. im Alkylthio-Teil, Dialkylaminoalkyl mit 1 bis 4 Kohlenstoffatomen in jedem Alkylteil, Halogenalkyl mit 1 bis 2 Kohlenstoff- und 1 bis 5 Halogenatomen, wobei als Halogene vorzugsweise Fluor und Chlor genannt seien, Alkoxycarbonylalkyl mit 1 bis 4 Kohlenstoffatomen im Alkylteil und 1 bis 4 Kohlenstoffatomen im Alkoxy-Teil sowie für die übrigen Reste, die für den Substituenten $R^6$ bereits oben genannt wurden. $R^5$ und $R^6$ stehen außerdem gemeinsam vorzugsweise für eine zwei- bis viergliedrige Alkylen-Gruppe.

Ganz besonders bevorzugt sind diejenigen Anilinomethyloxim-ether der Formel (I), in denen $R^1$, $R^2$ und $R^2$ gleich oder verschieden sind und für Wasserstoff, Methyl, Ethyl, Isopropyl, sek.-Butyl, tert.-Butyl, Methoxy, Ethoxy oder Isopropoxy stehen, $R^2$ und $R^3$ außerdem auch für Chlor oder Brom stehen ; $R^4$ für Wasserstoff, Methyl oder Ethyl steht ; $R^5$ für Wasserstoff, Methyl, Ethyl, Isopropyl sowie gegebenenfalls durch Chlor und/oder Methyl und/oder Methoxy und/oder Methylthio und/oder Trifluormethyl substituiertes Phenyl oder Naphthyl steht ; $R^6$ für Wasserstoff, Methyl, Ethyl, Isopropyl, n-Propyl, n-Butyl, tert.-Butyl, Vinyl, Allyl, Propargyl, Methoxymethyl, Ethoxymethyl, Ethoxyethyl, Methylthiomethyl, Chlormethyl, Chlorethyl, Dimethylaminomethyl, Ethyl-methylaminomethyl, Diethylaminomethyl, Methoxycarbonylmethyl, gegebenenfalls durch Chlor und/oder Methyl und/oder Methoxy und/oder Methylthio und/oder Trifluormethyl substituiertes Phenyl, Naphthyl und Benzyl steht ; und $R^5$ und $R^6$ außerdem gemeinsam für die Di- und Trimethylen-Gruppe stehen.

Als Beispiele für Verbindungen der Formel (I) seien im einzelnen die folgenden Verbindungen genannt :

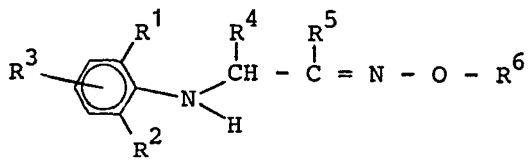

(I)

| $R^1$ | $R^2$ | $R^3$ | $R^4$ | $R^5$ | $R^6$ |
|---|---|---|---|---|---|
| H | H | H | H | H | H |
| $CH_3$ | $CH_3$ | H | H | H | H |
| $CH_3$ | $C_2H_5$ | H | H | H | H |
| $C_2H_5$ | $C_2H_5$ | H | H | H | H |
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |
| $C_2H_5$ | H | H | H | H | $CH_3$ |
| $C(CH_3)_3$ | H | H | H | H | $CH_3$ |
| $CH_3$ | Cl | H | H | H | $CH_3$ |
| $CH_3$ | $O-CH_3$ | H | H | H | $CH_3$ |
| $CH_3$ | Br | H | H | H | $CH_3$ |
| $C(CH_3)_3$ | Cl | H | H | H | $CH_3$ |
| $OCH_3$ | $OCH_3$ | H | H | H | $CH_3$ |
| $CH_3$ | H | $3-CH_3$ | H | H | $CH_3$ |
| $i-C_3H_7$ | $C_2H_5$ | H | H | H | $CH_3$ |
| $CH_3$ | H | H | H | H | $CH_3$ |
| $CH_3$ | $CH_3$ | H | H | H | $C_2H_5$ |
| $CH_3$ | $C_2H_5$ | H | H | H | $C_2H_5$ |
| $C_2H_5$ | $C_2H_5$ | H | H | H | $C_2H_5$ |

(Fortsetzung)

| $R^1$ | $R^2$ | $R^3$ | $R^4$ | $R^5$ | $R^6$ |
|---|---|---|---|---|---|
| $C_2H_5$ | H | H | H | H | $C_2H_5$ |
| $C(CH_3)_3$ | H | H | H | H | $C_2H_5$ |
| $CH_3$ | Cl | H | H | H | $C_2H_5$ |
| $CH_3$ | $O-CH_3$ | H | H | H | $C_2H_5$ |
| $CH_3$ | Br | H | H | H | $C_2H_5$ |
| $C(CH_3)_3$ | Cl | H | H | H | $C_2H_5$ |
| $OCH_3$ | $OCH_3$ | H | H | H | $C_2H_5$ |
| $CH_3$ | H | $3-CH_3$ | H | H | $C_2H_5$ |
| $i-C_3H_7$ | $C_2H_5$ | H | H | H | $C_2H_5$ |
| $CH_3$ | H | H | H | H | $C_2H_5$ |
| $CH_3$ | $CH_3$ | H | H | H | $n-C_3H_7$ |
| $CH_3$ | $C_2H_5$ | H | H | H | $n-C_3H_7$ |
| $C_2H_5$ | $C_2H_5$ | H | H | H | $n-C_3H_7$ |
| $C(CH_3)_3$ | H | H | H | H | $n-C_3H_7$ |
| $CH_3$ | $CH_3$ | H | H | H | $n-C_4H_9$ |
| $CH_3$ | $C_2H_5$ | H | H | H | $n-C_4H_9$ |
| $C_2H_5$ | $C_2H_5$ | H | H | H | $n-C_4H_9$ |
| $C(CH_3)_3$ | H | H | H | H | $n-C_4H_9$ |
| $CH_3$ | $CH_3$ | H | H | H | $t-C_4H_9$ |
| $CH_3$ | $C_2H_5$ | H | H | H | $t-C_4H_9$ |
| $C_2H_5$ | $C_2H_5$ | H | H | H | $t-C_4H_9$ |
| $C(CH_3)_3$ | H | H | H | H | $t-C_4H_9$ |
| $CH_3$ | $CH_3$ | H | H | H | $-CH_2-C\equiv CH$ |
| $CH_3$ | $C_2H_5$ | H | H | H | $-CH_2-C\equiv CH$ |
| $C_2H_5$ | $C_2H_5$ | H | H | H | $-CH_2-C\equiv CH$ |
| $C(CH_3)_3$ | H | H | H | H | $-CH_2-C\equiv CH$ |
| $CH_3$ | $CH_3$ | H | H | H | $-CH_2-CH=CH_2$ |
| $CH_3$ | $C_2H_5$ | H | H | H | $-CH_2-CH=CH_2$ |
| $C_2H_5$ | $C_2H_5$ | H | H | H | $-CH_2-CH=CH_2$ |
| $C(CH_3)_3$ | H | H | H | H | $-CH_2-CH=CH_2$ |
| $CH_3$ | $CH_3$ | H | H | H | $C_6H_5$ |
| $CH_3$ | $C_2H_5$ | H | H | H | $C_6H_5$ |
| $C_2H_5$ | $C_2H_5$ | H | H | H | $C_6H_5$ |
| $C(CH_3)_3$ | H | H | H | H | $C_6H_5$ |
| $CH_3$ | $CH_3$ | H | H | H | $-CH_2-O-CH_3$ |
| $CH_3$ | $C_2H_5$ | H | H | H | $-CH_2-O-CH_3$ |

(Fortsetzung)

| $R^1$ | $R^2$ | $R^3$ | $R^4$ | $R^5$ | $R^6$ |
|---|---|---|---|---|---|
| $C_2H_5$ | $C_2H_5$ | H | H | H | $-CH_2-O-CH_3$ |
| $C(CH_3)_3$ | H | H | H | H | $-CH_2-O-CH_3$ |
| $CH_3$ | $CH_3$ | H | H | H | $-CH_2-S-CH_3$ |
| $CH_3$ | $C_2H_5$ | H | H | H | $-CH_2-S-CH_3$ |
| $C_2H_5$ | $C_2H_5$ | H | H | H | $-CH_2-S-CH_3$ |
| $C(CH_3)_3$ | H | H | H | H | $-CH_2-S-CH_3$ |
| $CH_3$ | $CH_3$ | H | H | H | $-CH_2-C_6H_5$ |
| $CH_3$ | $C_2H_5$ | H | H | H | $-CH_2-C_6H_5$ |
| $C_2H_5$ | $C_2H_5$ | H | H | H | $-CH_2-C_6H_5$ |
| $C(CH_3)_3$ | H | H | H | H | $-CH_2-C_6H_5$ |
| $CH_3$ | $CH_3$ | H | H | H | $-CH_2-CH_2Cl$ |
| $CH_3$ | $C_2H_5$ | H | H | H | $-CH_2-CH_2Cl$ |
| $C_2H_5$ | $C_2H_5$ | H | H | H | $-CH_2-CH_2Cl$ |
| $C(CH_3)_3$ | H | H | H | H | $-CH_2-CH_2Cl$ |
| $CH_3$ | $CH_3$ | H | H | $CH_3$ | $n-C_3H_7$ |
| $CH_3$ | $C_2H_5$ | H | H | $CH_3$ | $n-C_3H_7$ |
| $C_2H_5$ | $C_2H_5$ | H | H | $CH_3$ | $n-C_3H_7$ |
| $C(CH_3)_3$ | H | H | H | $CH_3$ | $n-C_3H_7$ |
| $CH_3$ | $CH_3$ | H | H | $CH_3$ | $n-C_4H_9$ |
| $CH_3$ | $C_2H_5$ | H | H | $CH_3$ | $n-C_4H_9$ |
| $C_2H_5$ | $C_2H_5$ | H | H | $CH_3$ | $n-C_4H_9$ |
| $C(CH_3)_3$ | H | H | H | $CH_3$ | $n-C_4H_9$ |
| $CH_3$ | $CH_3$ | H | H | $CH_3$ | $t-C_4H_9$ |
| $CH_3$ | $C_2H_5$ | H | H | $CH_3$ | $t-C_4H_9$ |
| $C_2H_5$ | $C_2H_5$ | H | H | $CH_3$ | $t-C_4H_9$ |
| $C(CH_3)_3$ | H | H | H | $CH_3$ | $t-C_4H_9$ |
| $CH_3$ | $CH_3$ | H | H | $CH_3$ | $-CH_2-C\equiv CH$ |
| $CH_3$ | $C_2H_5$ | H | H | $CH_3$ | $-CH_2-C\equiv CH$ |
| $C_2H_5$ | $C_2H_5$ | H | H | $CH_3$ | $-CH_2-C\equiv CH$ |
| $C(CH_3)_3$ | H | H | H | $CH_3$ | $-CH_2-C\equiv CH$ |
| $CH_3$ | $CH_3$ | H | H | $CH_3$ | $-CH_2-CH=CH_2$ |
| $CH_3$ | $C_2H_5$ | H | H | $CH_3$ | $-CH_2-CH=CH_2$ |
| $C_2H_5$ | $C_2H_5$ | H | H | $CH_3$ | $-CH_2-CH=CH_2$ |
| $C(CH_3)_3$ | H | H | H | $CH_3$ | $-CH_2-CH=CH_2$ |
| $CH_3$ | $CH_3$ | H | H | $CH_3$ | $C_6H_5$ |
| $CH_3$ | $C_2H_5$ | H | H | $CH_3$ | $C_6H_5$ |

(Fortsetzung)

| $R^1$ | $R^2$ | $R^3$ | $R^4$ | $R^5$ | $R^6$ |
|---|---|---|---|---|---|
| $C_2H_5$ | $C_2H_5$ | H | H | $CH_3$ | $C_6H_5$ |
| $C(CH_3)_3$ | H | H | H | $CH_3$ | $C_6H_5$ |
| $CH_3$ | $CH_3$ | H | H | $CH_3$ | $-CH_2-O-CH_3$ |
| $CH_3$ | $C_2H_5$ | H | H | $CH_3$ | $-CH_2-O-CH_3$ |
| $C_2H_5$ | $C_2H_5$ | H | H | $CH_3$ | $-CH_2-O-CH_3$ |
| $C(CH_3)_3$ | H | H | H | $CH_3$ | $-CH_2-O-CH_3$ |
| $CH_3$ | $CH_3$ | H | H | $CH_3$ | $-CH_2-S-CH_3$ |
| $CH_3$ | $C_2H_5$ | H | H | $CH_3$ | $-CH_2-S-CH_3$ |
| $C_2H_5$ | $C_2H_5$ | H | H | $CH_3$ | $-CH_2-S-CH_3$ |
| $C(CH_3)_3$ | H | H | H | $CH_3$ | $-CH_2-S-CH_3$ |
| $CH_3$ | $CH_3$ | H | H | $CH_3$ | $-CH_2-C_6H_5$ |
| $CH_3$ | $C_2H_5$ | H | H | $CH_3$ | $-CH_2-C_6H_5$ |
| $C_2H_5$ | $C_2H_5$ | H | H | $CH_3$ | $-CH_2-C_6H_5$ |
| $C(CH_3)_3$ | H | H | H | $CH_3$ | $-CH_2-C_6H_5$ |
| $CH_3$ | $CH_3$ | H | H | $CH_3$ | $-CH_2-CH_2-Cl$ |
| $CH_3$ | $C_2H_5$ | H | H | $CH_3$ | $-CH_2-CH_2-Cl$ |
| $C_2H_5$ | $C_2H_5$ | H | H | $CH_3$ | $-CH_2-CH_2-Cl$ |
| $C(CH_3)_3$ | H | H | H | $CH_3$ | $-CH_2-CH_2-Cl$ |
| $CH_3$ | $CH_3$ | H | H | $-(CH_2)_2-$ | |
| $CH_3$ | $C_2H_5$ | H | H | $-(CH_2)_2-$ | |
| $C_2H_5$ | $C_2H_5$ | H | H | $-(CH_2)_2-$ | |
| $C(CH_3)_3$ | H | H | H | $-(CH_2)_2-$ | |
| $CH_3$ | $CH_3$ | H | H | $-(CH_2)_3-$ | |
| $CH_3$ | $C_2H_5$ | H | H | $-(CH_2)_3-$ | |
| $C_2H_5$ | $C_2H_5$ | H | H | $-(CH_2)_3-$ | |
| $C(CH_3)_3$ | H | H | H | $-(CH_2)_3-$ | |
| $CH_3$ | $CH_3$ | H | $CH_3$ | $-(CH_2)_2-$ | |
| $CH_3$ | $C_2H_5$ | H | $CH_3$ | $-(CH_2)_2-$ | |
| $C_2H_5$ | $C_2H_5$ | H | $CH_3$ | $-(CH_2)_2-$ | |
| $C(CH_3)_3$ | H | H | $CH_3$ | $-(CH_2)_2-$ | |
| $CH_3$ | $CH_3$ | H | $CH_3$ | $-(CH_2)_3-$ | |
| $CH_3$ | $C_2H_5$ | H | $CH_3$ | $-(CH_2)_3-$ | |
| $C_2H_5$ | $C_2H_5$ | H | $CH_3$ | $-(CH_2)_3-$ | |
| $C(CH_3)_3$ | H | H | $CH_3$ | $-(CH_2)_3-$ | |
| $CH_3$ | $CH_3$ | H | H | $C_2H_5$ | H |
| $CH_3$ | $C_2H_5$ | H | H | $C_2H_5$ | H |
| $C_2H_5$ | $C_2H_5$ | H | H | $C_2H_5$ | H |

(Fortsetzung)

| $R^1$ | $R^2$ | $R^3$ | $R^4$ | $R^5$ | $R^6$ |
|---|---|---|---|---|---|
| $C(CH_3)_3$ | H | H | H | $C_2H_5$ | H |
| $CH_3$ | $CH_3$ | H | H | $C_2H_5$ | $CH_3$ |
| $CH_3$ | $C_2H_5$ | H | H | $C_2H_5$ | $CH_3$ |
| $C_2H_5$ | $C_2H_5$ | H | H | $C_2H_5$ | $CH_3$ |
| $C(CH_3)_3$ | H | H | H | $C_2H_5$ | $CH_3$ |
| $CH_3$ | $CH_3$ | H | H | $C_2H_5$ | $C_2H_5$ |
| $CH_3$ | $C_2H_5$ | H | H | $C_2H_5$ | $C_2H_5$ |
| $C_2H_5$ | $C_2H_5$ | H | H | $C_2H_5$ | $C_2H_5$ |
| $C(CH_3)_3$ | H | H | H | $C_2H_5$ | $C_2H_5$ |
| $CH_3$ | $CH_3$ | H | H | $C_6H_5$ | H |
| $CH_3$ | $C_2H_5$ | H | H | $C_6H_5$ | H |
| $C_2H_5$ | $C_2H_5$ | H | H | $C_6H_5$ | H |
| $CH_3$ | $CH_3$ | H | H | $C_6H_5$ | $CH_3$ |
| $CH_3$ | $C_2H_5$ | H | H | $C_6H_5$ | $CH_3$ |
| $C_2H_5$ | $C_2H_5$ | H | H | $C_6H_5$ | $CH_3$ |
| $C(CH_3)_3$ | H | H | H | $C_6H_5$ | $CH_3$ |
| $CH_3$ | Cl | H | H | $C_6H_5$ | $CH_3$ |
| $CH_3$ | Br | H | H | $C_6H_5$ | $CH_3$ |
| $OCH_3$ | $CH_3$ | H | H | $C_6H_5$ | $CH_3$ |
| $CH_3$ | H | H | H | $C_6H_5$ | $CH_3$ |
| $CH_3$ | H | 3-CH | H | $C_6H_5$ | $CH_3$ |
| $CH_3$ | H | 5-$CH_3$ | H | $C_6H_5$ | $CH_3$ |
| $CH_3$ | $CH_3$ | H | H | $C_6H_5$ | $C_2H_5$ |
| $CH_3$ | $C_2H_5$ | H | H | $C_6H_5$ | $C_2H_5$ |
| $C_2H_5$ | $C_2H_5$ | H | H | $C_6H_5$ | $C_2H_5$ |
| $C(CH_3)_3$ | H | H | H | $C_6H_5$ | $C_2H_5$ |
| $CH_3$ | $CH_3$ | H | H | $C_6H_5$ | n-$C_3H_7$ |
| $CH_3$ | $C_2H_5$ | H | H | $C_6H_5$ | n-$C_3H_7$ |
| $C_2H_5$ | $C_2H_5$ | H | H | $C_6H_5$ | n-$C_3H_7$ |
| $C(CH_3)_3$ | H | H | H | $C_6H_5$ | n-$C_3H_7$ |
| $CH_3$ | $CH_3$ | H | $CH_3$ | H | $CH_3$ |
| $CH_3$ | $C_2H_5$ | H | $CH_3$ | H | $CH_3$ |
| $C_2H_5$ | $C_2H_5$ | H | $CH_3$ | H | $CH_3$ |
| $C(CH_3)_3$ | H | H | $CH_3$ | H | $CH_3$ |
| $CH_3$ | $CH_3$ | H | $CH_3$ | $CH_3$ | $CH_3$ |
| $CH_3$ | $C_2H_5$ | H | $CH_3$ | $CH_3$ | $CH_3$ |

(Fortsetzung)

| $R^1$ | $R^2$ | $R^3$ | $R^4$ | $R^5$ | $R^6$ |
|---|---|---|---|---|---|
| $C_2H_5$ | $C_2H_5$ | H | $CH_3$ | $CH_3$ | $CH_3$ |
| $C(CH_3)_3$ | H | H | $CH_3$ | $CH_3$ | $CH_3$ |
| $CH_3$ | $CH_3$ | H | $CH_3$ | H | $C_2H_5$ |
| $CH_3$ | $C_2H_5$ | H | $CH_3$ | H | $C_2H_5$ |
| $C_2H_5$ | $C_2H_5$ | H | $CH_3$ | H | $C_2H_5$ |
| $C(CH_3)_3$ | H | H | $CH_3$ | H | $C_2H_5$ |
| $CH_3$ | $CH_3$ | H | $CH_3$ | $CH_3$ | $C_2H_5$ |
| $CH_3$ | $C_2H_5$ | H | $CH_3$ | $CH_3$ | $C_2H_5$ |
| $C_2H_5$ | $C_2H_5$ | H | $CH_3$ | $CH_3$ | $C_2H_5$ |
| $C(CH_3)_3$ | H | H | $CH_3$ | $CH_3$ | $C_2H_5$ |
| $CH_3$ | $CH_3$ | H | $CH_3$ | H | $n-C_3H_7$ |
| $CH_3$ | $C_2H_5$ | H | $CH_3$ | H | $n-C_3H_7$ |
| $C_2H_5$ | $C_2H_5$ | H | $CH_3$ | H | $n-C_3H_7$ |
| $C(CH_3)_3$ | H | H | $CH_3$ | H | $n-C_3H_7$ |
| $CH_3$ | $CH_3$ | H | $CH_3$ | $CH_3$ | $n-C_3H_7$ |
| $CH_3$ | $C_2H_5$ | H | $CH_3$ | $CH_3$ | $n-C_3H_7$ |
| $C_2H_5$ | $C_2H_5$ | H | $CH_3$ | $CH_3$ | $n-C_3H_7$ |
| $C(CH_3)_3$ | H | H | $CH_3$ | $CH_3$ | $n-C_3H_7$ |
| $CH_3$ | $CH_3$ | H | $CH_3$ | H | $n-C_4H_9$ |
| $CH_3$ | $C_2H_5$ | H | $CH_3$ | H | $n-C_4H_9$ |
| $C_2H_5$ | $C_2H_5$ | H | $CH_3$ | H | $n-C_4H_9$ |
| $C(CH_3)_3$ | H | H | $CH_3$ | H | $n-C_4H_9$ |

Die bei der Herstellung der Anilinomethyloxim-ether der Formel (I) als Ausgangsstoffe benötigten Aniline der Formel (II) sind allgemein bekannte Verbindungen der organischen Chemie. Als Beispiele seien genannt :

Anilin ; 2-Methylanilin ; 2-Ethylanilin ; 2-Isopropylanilin ; 2-sek.-Butylanilin ; 2-tert.-Butylanilin ; 2,6-Dimethylanilin ; 2,3-Dimethylanilin ; 2,5-Dimethylanilin ; 3,5-Dimethylanilin ; 2,6-Diethylanilin ; 2-Ethyl-6-methylanilin ; 2,3,4-Trimethylanilin ; 2,4,6-Trimethylanilin ; 2,4,5-Trimethylanilin ; 2-Ethyl-4,6-dimethylanilin ; 2,6-Diethyl-4-methylanilin ; 2,6-Diisopropyl-4-methylanilin ; 2,3,5-Trimethylanilin ; 2,3,6-Trimethylanilin ; 2-Methyl-6-chloranilin ; 2-tert.-Butyl-6-chloranilin ; 2-Methoxy-6-methylanilin ; 2,6-Dimethoxyanilin ; 2-Methoxy-6-ethylanilin ; 2,6-Diethoxyanilin.

Die bei der Herstellung der Anilinomethyloxim-ether der Formel (I) nach dem Verfahren (a) (Variante α) weiterhin als Ausgangsstoffe benötigten substituierten Oximether der Formel (III) sind bekannt (vgl. z. B. US-Patentschrift 3 896 189) ; ebenso die für das Verfahren (a), Variante (β) benötigten Propargylhalogenide der Formel (IV) (vgl.Houben-Weyl, Methoden der organischen Chemie, Band V/3 und 4) ; sowie auch die für das Verfahren (a), Variante (γ) benötigten Keto-Derivate der Formel (VIII) (vgl.Houben-Weyl, Methoden der organischen Chemie, Band VII/2 a,b,c) und die Hydroxylamin-(Derivate) der Formel (VII) (vgl.Houben-Weyl, Methoden der organischen Chemie, Band X/1) ; bzw. lassen sich die jeweiligen Verbindungen nach den in den erwähnten Literaturstellen angegebenen Verfahren leicht herstellen.

Bei der Herstellung der Anilinomethyloxim-ether der Formel (I) nach dem Verfahren (a) Varianten (α), (β) und (γ) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei dem Verfahren (a), Varianten (α), (β) und (γ) alle üblichen inerten organischen Lösungsmittel eingesetzt werden. Vorzugsweise in Betracht kommen aromatische Lösungsmittel, wie insbesondere Toluol, sowie Dimethylformamid. In der zweiten Stufe des Verfahrens (a), Variante (γ) kommen vorzugsweise Alkohole, wie insbesondere Methanol, als Lösungsmittel in Frage.

Die Reaktionstemperaturen können bei dem Verfahren (a), Varianten (α), (β) und (γ) innerhalb eines größeren Bereiches variiert werden. Im allgemeinen arbeitet man zwischen 0 °C und 180 °C, vorzugsweise zwischen 20 °C und 160 °C.

Bei der Umsetzung gemäß Verfahren (a), Varianten (α), (β) und (γ) arbeitet man vorzugsweise mit äquimolaren Mengen. Es ist jedoch auch möglich, eine der Komponenten, vorzugsweise das Anilin der Formel (II), in einem Überschuß einzusetzen. Die Aufarbeitung und Isolierung der Reaktionsprodukte erfolgt nach üblichen Methoden.

Die bei der Durchführung des Verfahrens (b) als Ausgangsstoffe benötigten Oxime der Formel (X) sind bisher noch nicht bekannt. Sie lassen sich jedoch in einfacher Weise nach dem Verfahren (a), Variante (γ) herstellen.

Die bei dem Verfahren (b) weiterhin als Ausgangsstoffe benötigten Halogenide sind durch die Formel (XI) allgemein definiert. In dieser Formel steht $R^7$ vorzugsweise für geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils 1 bis 4 Kohlenstoffatomen im Alkylteil und 1 bis 4 Kohlenstoffatomen im Alkoxy- bzw. im Alkylthio-Teil, Dialkylaminoalkyl mit 1 bis 4 Kohlenstoffatomen in jedem Alkylteil, Halogenalkyl mit 1 bis 2 Kohlenstoffatomen und 1 bis 5 Halogenatomen, wobei als Halogene vorzugsweise Fluor und Chlor genannt seien, Alkoxycarbonylalkyl mit 1 bis 4 Kohlenstoffatomen im Alkylteil und 1 bis 4 Kohlenstoffatomen im Alkoxyteil sowie für die übrigen Reste, die für den Substituenten $R^7$ bereits oben genannt wurden.

Bei dem Verfahren (b) kommen als Verdünnungsmittel alle inerten organischen Lösungsmittel in Frage. Hierzu gehören vorzugsweise Ether, wie Diethylether und Dioxan ; aromatische Kohlenwasserstoffe, wie Toluol und Benzol ; in einzelnen Fällen auch chlorierte Kohlenwasserstoffe, wie Chloroform, Methylenchlorid und Tetrachlorkohlenstoff ; sowie Hexamethylphosphorsäuretriamid.

Die Reaktionstemperaturen können bei der Durchführung des Verfahrens (b) in einem größeren Bereich variiert werden. Im allgemeinen arbeitet man zwischen 20 und 150 °C, vorzugsweise bei Raumtemperatur. In einzelnen Fällen ist es vorteilhaft, bei der Siedetemperatur des Lösungsmittels, beispielsweise zwischen 60 und 100 °C, zu arbeiten.

Bei der Durchführung des Verfahrens (b) setzt man auf 1 Mol Alkalisalz eines Oxims der Formel (X) vorzugsweise 1 bis 3 Mol Halogenid der Formel (XI) ein. Die Aufarbeitung und Isolierung der Reaktionsprodukte erfolgt nach üblichen Methoden.

In einer bevorzugten Ausführungsform des Verfahrens (b) wird zweckmäßigerweise so verfahren, daß man von einem Oxim der Formel (X) ausgeht, letzteres in einem geeigneten inerten organischen Lösungsmittel mittels Alkalimetall-hydrid oder -amid in das Salz überführt, und letzteres ohne Isolierung sofort mit einem Halogenid der Formel (XI) umsetzt, wobei unter Austritt von Alkalihalogenid die Verbindungen der Formel (I) in einem Arbeitsgang erhalten werden.

Nach einer weiteren bevorzugten Ausführungsform des Verfahrens (b) werden zweckmäßigerweise die Herstellung der Salze der Oxime der Formel (X) sowie die Umsetzung in einem Zweiphasensystem, wie beispielsweise wäßrige Natron- und Kalilauge/Toluol oder Methylenchlorid, unter Zusatz von 0,01 — 1 Mol eines Phase-Transfer-Katalysators, wie beispielsweise Ammonium- oder Phosphoniumverbindungen, durchgeführt.

Die erfindungsgemäßen Anilinomethyloximether der Formel (I) eignen sich als Zwischenprodukte zur Synthese von Oximethern, welche herbizide Eigenschaften besitzen. Diese herbizid wirksamen Oximether der Formel

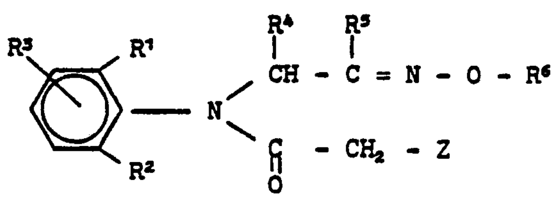

(XII)

in welcher

$R^1$, $R^2$, $R^3$, $R^4$, $R^5$ und $R^6$ die oben angegebene Bedeutung haben und

Z für Halogen steht, lassen sich herstellen, indem man Anilinomethyloxim-ether der Formel

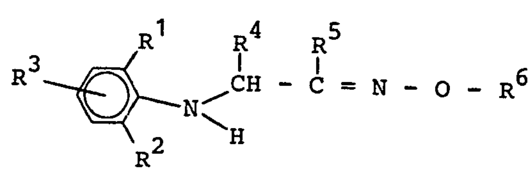

(I)

in welcher

$R^1$, $R^2$, $R^3$, $R^4$ und $R^6$ die oben angegebene Bedeutung haben, mit Halogenessigsäurechloriden oder -bromiden bzw. -anhydriden der Formel

$$Z—CH_2—CO—Cl(Br) \quad (XIIIa)$$

bzw.

$$(Z—CH_2—CO)_2O \quad (XIIIb)$$

in welchen

Z die oben angegebene Bedeutung hat, in Gegenwart eines Verdünnungsmittels und gegebenenfalls eines Säurebindemittels umsetzt.

Die bei dieser Umsetzung als Ausgangsstoffe zu verwendenden Halogenessigsäurechloride und -bromide bzw. -anhydride sind durch die Formeln (XIIIa) und (XIIIb) allgemein definiert. In diesen Formeln steht *Z* vorzugsweise für Chlor, Brom und Jod.

Die Halogenessigsäurechloride und -bromide bzw. -anhydride der Formeln (XIIIa) und (XIIIb) sind allgemein bekannte Verbindungen der organischen Chemie. Als Beispiele seien genannt : Chloracetylchlorid, Bromacetylchlorid, Jodacetylchlorid und die entsprechenden Bromide sowie Anhydride.

Für die Umsetzung der erfindungsgemäßen Anilinomethyloximether der Formel (I) mit Komponenten der Formeln (XIIIa) oder (XIIIb) kommen als Verdünnungsmittel vorzugsweise inerte organische Lösungsmittel in Frage. Hierzu gehören vorzugsweise Ketone, wie Diethylketon, insbesondere Aceton und Methylethylketon ; Nitrile, wie Propionitril, insbesondere Acetonitril ; Ether, wie Tetrahydrofuran oder Dioxan ; aliphatische und aromatische Kohlenwasserstoffe, wie Petrolether, Benzol, Toluol oder Xylol ; halogenierte Kohlenwasserstoffe, wie Methylenchlorid, Tetrachlorkohlenstoff, Chloroform oder Chlorbenzol ; und Ester, wie Essigester.

Das oben angegebene Verfahren zur Herstellung von Oximethern der Formel (XII) kann in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des oben angegebenen Verfahrens in einem größeren Bereich variiert werden. Im allgemeinen arbeitet man zwischen 0 °C und 120 °C, vorzugsweise zwischen 20 und 100 °C.

Bei der Durchführung des oben angegebenen Verfahrens setzt man vorzugsweise auf 1 Mol der Verbindungen der Formel (I) 1 bis 1,5 Mol Halogenacetylierungsmittel und 1 bis 1,5 Mol Säurebinder ein. Die Isolierung der Verbindungen der Formel (XII) erfolgt in üblicher Weise.

Die Oximether der Formel (XII) beeinflussen das Pflanzenwachstum und können deshalb als Defoliants, Desiccants, Krautabtötungsmittel, Keimhemmungsmittel und insbesondere als Unkrautvernichtungsmittel verwendet werden.

Sie zeigen vor allem neben einer sehr guten Wirkung gegen grasartige Unkräuter und gegen Cyperus-Arten auch eine gute herbizide Wirkung bei breitblättrigen Unkräutern. Ein selektiver Einsatz der Wirkstoffe der Formel (XII) ist möglich, vorzugsweise in Getreide, Baumwolle und Zuckerrüben, oder auch in anderen Kulturen.

Die guten herbiziden Wirkungen der Oximether der Formel (XII) gehen aus dem nachstehend beschriebenen Verwendungsbeispiel hervor.

Wirkstoffliste

In dem nachfolgenden Beispiel werden die nachstehend angegebenen Wirkstoffe bezüglich ihrer herbiziden Wirkung getestet.

A = 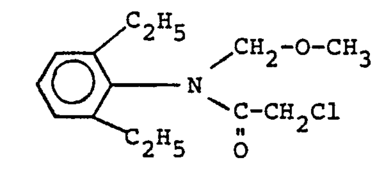 (bekannt)

2,6-Diethyl-N-methoxymethyl-chloracetanilid

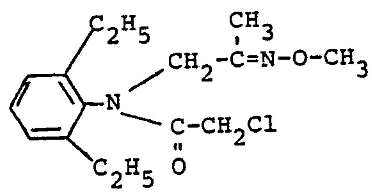

XII-3

Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Wirkstoffe, Aufwandmengen und Resultate werden ermittelt.

Der Oximether der Formel XII-3 zeigt in diesem Test eine bessere selektive Wirksamkeit als die aus dem Stand der Technik bekannte Substanz (A).

Herstellungsbeispiele

Beispiel 1

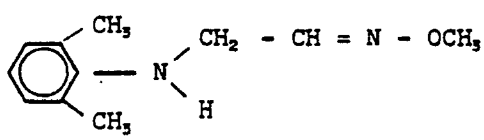

(Verfahren a), Variante (α)

48,4 g (0,4 Mol) 2,6-Dimethylanilin und 27,6 g (0,2 Mol) fein gepulvertes Kaliumcarbonat werden in 80 ml Dimethylformamid unter Rühren auf 100 °C erhitzt und tropfenweise mit 21,5 g (0,2 Mol) Chloracetaldehyd-O-methyl-oximether versetzt. Nach 3 Stunden Rühren bei 100 °C wird das anorganische Salz abfiltriert. Das Filtrat wird mit 250 ml Methylenchlorid versetzt, mehrmals mit Wasser ausgeschüttelt, über Natriumsulfat getrocknet und im Vakuum eingeengt. Der Rückstand wird destilliert. Man erhält 14,2 g (37 % der Theorie) 2,6-Dimethyl-N-(2'-methoxy-imino-ethyl)-anilin vom Siedepunkt 105-110 °C/0,133 mbar und vom Brechungsindex $n_D^{20}$ : 1,537.

Umsetzung der Verbindung (1) zu dem entsprechenden Oximether der Formel

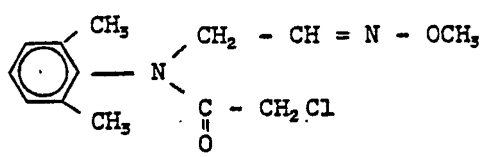

(XII-1)

6 g (0,03 Mol) 2,6-Dimethyl-N-(2'-methoxyimino-ethyl)-anilin und 2,8 g (0,035 Mol) Pyridin werden in 80 ml wasserfreiem Tetrahydrofuran zum Sieden erhitzt und mit 4 g (0,035 Mol) Chloracetylchlorid versetzt. Nach 15 Minuten Erhitzen unter Rückfluß wird die Reaktionsmischung abgekühlt und im Vakuum eingeengt. Der Rückstand wird zwischen Wasser und Methylenchlorid verteilt, die organische

Phase abgetrennt, über Natriumsulfat getrocknet und eingeengt. Der ölige Rückstand kristallisiert nach Behandeln mit Petrolether. Man erhält 4,8 g (60 % der Theorie) 2,6-Dimethyl-N-(2′-methoxyimino-ethyl)-chloracetanilid vom Schmelzpunkt 56-58 °C.

Beispiel 2

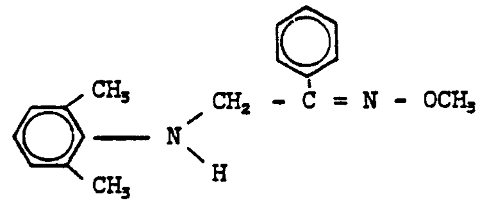

(Verfahren b)

31,2 g (0,11 Mol) α-(2,6-Dimethylphenylamino)acetophenonoxim-hydrochlorid und 0,6 g (0,176 Mol) Benzyldimethyl-ammoniumchlorid werden in 110 ml Methylenchlorid gelöst und nach Zugabe von 110 ml konzentrierter Natronlauge und 6,9 g (0,055 Mol) Dimethylsulfat 6 Tage bei 20 °C gerührt. Nach Aufgießen auf Eis wird die organische Phase abgetrennt, mit Wasser gewaschen, über Natriumsulfat getrocknet und eingedampft. Man erhält 25,7 g (87 % der Theorie) α-(2,6-Dimethylphenylamino)-acetophenonoxim-O-methylether vom Brechungsindex $n_D^{20}$ : 1,587 7.

Umsetzung der Verbindung (2) zu dem entsprechenden Oximether der Formel

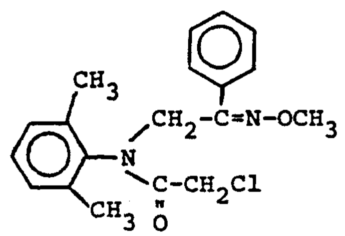

(XII-2)

13,4 g (0,05 Mol) α-(2,6-Dimethylphenylamino)-acetophenonoxim-O-methylether werden in 200 ml Toluol gelöst, mit 4 ml (0,05 Mol) Chloracetylchlorid versetzt und 6 Stunden bis zur beendeten Gasentwicklung unter Rückfluß erhitzt. Man läßt abkühlen und engt durch Abdestillieren des Lösungsmittels im Vakuum ein. Der Rückstand wird chromatographisch gereinigt. Man erhält 3,1 g (18 % der Theorie) α-[2,6-Dimethylphenyl)-N-chloracetyl-amino]-acetophenonoxim-O-methylether vom Schmelzpunkt 80 °C.

Beispiel 3

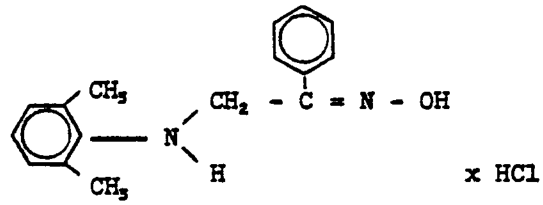

(Verfahren (a) Variante (γ))

59,7 g (0,3 Mol) Phenacylbromid (Herstellung vgl. Org. Synthesis Vol. II, S.480) und 73,2 g (0,6 Mol) 2,6-Dimethylanilin werden in 100 ml Ethylalkohol 10 Minuten auf 80 °C erhitzt und danach im Vakuum eingeengt. Der Rückstand wird in Toluol aufgenommen und filtriert. Das Filtrat wird mit Ethylalkohol verdünnt und mit 20,8 g (0,165 Mol) Hydroxylammoniumchlorid sowie mit 22,6 g (0,165 Mol) gepulvertem Kaliumcarbonat versetzt. Nach 20-stündigem Rühren bei Raumtemperatur wird filtriert und das Filtrat eingeengt. Der Rückstand wird durch Verreiben mit wenig Ethylalkohol zur Kristallisation gebracht. Man erhält 33,9 g (40 % der Theorie) α-(2,6-Dimethylphenylamino)-aceto-phenonoxim-hydrochlorid vom Schmelzpunkt 200 °C.

Nach einem oder mehreren der in der Anmeldung beschriebenen Verfahren und entsprechend den Beispielen 1 bis 3 werden die in der nachstehenden Tabelle 1 formelmäßig aufgeführten erfindungsgemäßen Verbindungen der Formel (I) erhalten.

Tabelle 1

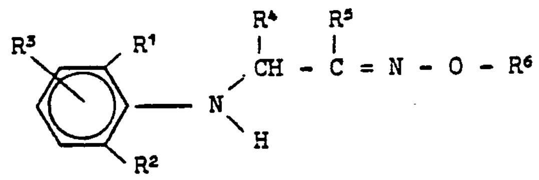

(I)

| Bsp. Nr. | $R^1$ | $R^2$ | $R^3$ | $R^4$ | $R^5$ | $R^6$ | Kp(°C)/mbar bzw. $n_D^{20}$ |
|---|---|---|---|---|---|---|---|
| 4 | $C_2H_5$ | $CH_3$ | H | H | H | $CH_3$ | 120-25/0,133<br>1,531 |
| 5 | $C_2H_5$ | $C_2H_5$ | H | H | H | $CH_3$ | 112-17/0,133<br>1,5295 |
| 6 | $CH_3$ | $C_2H_5$ | H | H | H | $C_2H_5$ | 120-21/0,133<br>1,525 |
| 7 | $C_2H_5$ | $C_2H_5$ | H | H | H | $C_2H_5$ | 135-40/0,133<br>1,525 |
| 8 | $C_2H_5$ | $C_2H_5$ | H | H | H | n-$C_4H_9$ | 120-35/0,133<br>1,513 |

Nach dem in der Anmeldung beschriebenen Verfahren und entsprechend den Beispielen 1 und 2 lassen sich aus den Anilinomethyloxim-ethern der Formel (I) die Oximether der Formel (XII) herstellen, die in der folgenden Tabelle 2 formelmäßig aufgeführt sind.

Tabelle 2

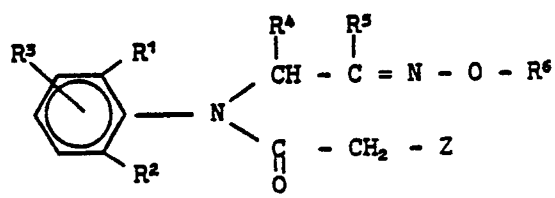

(XII)

| Bsp. Nr. | $R^1$ | $R^2$ | $R^3$ | $R^4$ | $R^5$ | $R^6$ | Z | Schmelzpunkt(°C) bzw. Brechungsindex |
|---|---|---|---|---|---|---|---|---|
| XII-3 | $C_2H_5$ | $C_2H_5$ | H | H | $CH_3$ | $CH_3$ | Cl | 56-57 |
| XII-4 | $CH_3$ | $CH_3$ | H | H | $CH_3$ | $CH_3$ | Cl | 61-75 |
| XII-5 | $CH_3$ | $C_2H_5$ | H | H | $CH_3$ | $CH_3$ | Cl | 53 |
| XII-6 | $CH_3$ | $CH_3$ | H | H | $CH_3$ | $C_2H_5$ | Cl | 70-82 |
| XII-7 | $C_2H_5$ | $CH_3$ | H | H | $CH_3$ | $C_2H_5$ | Cl | 62 |
| XII-8 | $C_2H_5$ | $C_2H_5$ | H | H | $CH_3$ | n-$C_4H_9$ | Cl | $n_D^{22}$:1,520 |
| XII-9 | $CH_3$ | $C_2H_5$ | H | H | $CH_3$ | n-$C_4H_9$ | Cl | $n_D^{22}$:1,523 |
| XII-10 | $C_2H_5$ | $C_2H_5$ | H | H | H | $CH_3$ | Cl | 49-50 |
| XII-11 | $CH_3$ | $C_2H_5$ | H | H | H | $CH_3$ | Cl | $n_D^{22}$:1,541 |
| XII-12 | $C_2H_5$ | $C_2H_5$ | H | H | H | $C_2H_5$ | Cl | Kristallbrei |

(Fortsetzung)

| Bsp. Nr. | $R^1$ | $R^2$ | $R^3$ | $R^4$ | $R^5$ | $R^6$ | Z | Schmelzpunkt (°C) bzw. Brechungsindex |
|---|---|---|---|---|---|---|---|---|
| XII-13 | $CH_3$ | $C_2H_5$ | H | H | H | $C_2H_5$ | Cl | $n_D^{23}$:1,5336 |
| XII-14 | $C_2H_5$ | $C_2H_5$ | H | H | $CH_3$ | H | Cl | 139-40 |
| XII-15 | $CH_3$ | $C_2H_5$ | H | H | $CH_3$ | H | Cl | 111-13 |
| XII-16 | $C_2H_5$ | $C_2H_5$ | H | H | H | n-$C_4H_9$ | Cl | $n_D^{23}$:1,521 |
| XII-17 | $CH_3$ | Cl | H | H | $CH_3$ | $CH_3$ | Cl | 77-78 |
| XII-18 | H | $CH_3$ | H | H | $CH_3$ | $CH_3$ | Cl | $n_D^{20}$=1,5402 |
| XII-19 | H | $C(CH_3)_3$ | H | H | $CH_3$ | $CH_3$ | Cl | 95-101 |
| XII-20 | $CH_3$ | $CH_3$ | H | H | $CH_3$ | $C_3H_7$ | Cl | Kristallbrei |
| XII-21 | $CH_3$ | H | 3-$CH_3$ | H | $CH_3$ | $CH_3$ | Cl | 48-52 |
| XII-22 | $C_2H_5$ | $C_2H_5$ | H | H | $CH_3$ | $C_2H_5$ | Cl | $n_D^{20}$=1,5282 |
| XII-23 | $CH_3$ | $C_2H_5$ | H | H | $CH_3$ | $C_3H_7$ | Cl | $n_D^{20}$=1,5276 |
| XII-24 | $CH_3$ | $CH_3$ | H | H | $CH_3$ | H | Cl | 123-125 |
| XII-25 | $CH_3$ | $CH_3$ | H | $CH_3$ | $CH_3$ | $CH_3$ | Cl | $n_D^{20}$=1,5388 |
| XII-26 | $CH_3$ | $CH_3$ | H | $CH_3$ | H | $CH_3$ | Cl | $n_D^{20}$=1,5398 |
| XII-27 | $CH_3$ | $CH_3$ | H | H | H | $C_2H_5$ | Cl | $n_D^{20}$=1,5369 |
| XII-28 | $CH_3$ | $C_2H_5$ | H | $CH_3$ | $CH_3$ | $CH_3$ | Cl | $n_D^{20}$=1,5298 |
| XII-29 | $C_2H_5$ | $C_2H_5$ | H | H | $CH_3$ | $C_3H_7$ | Cl | $n_D^{20}$=1,5262 |
| XII-30 | $C_2H_5$ | $C_2H_5$ | H | $CH_3$ | $CH_3$ | $CH_3$ | Cl | $n_D^{20}$=1,5300 |
| XII-31 | $CH_3$ | $CH_3$ | H | H | H | $C_3H_7$ | Cl | $n_D^{20}$=1,5312 |
| XII-32 | $CH_3$ | $C_2H_5$ | H | H | H | $C_3H_7$ | Cl | $n_D^{20}$=1,5155 |
| XII-33 | $C_2H_5$ | $C_2H_5$ | H | H | H | $C_3H_7$ | Cl | $n_D^{20}$=1,5209 |
| XII-34 | $CH_3$ | $CH_3$ | H | $CH_3$ | H | $C_2H_5$ | Cl | $n_D^{20}$=1,5348 |
| XII-35 | $CH_3$ | $C_2H_5$ | H | $CH_3$ | H | $CH_3$ | Cl | Kristallbrei |
| XII-36 | $CH_3$ | $C_2H_5$ | H | $CH_3$ | H | $C_2H_5$ | Cl | $n_D^{20}$=1,5290 |
| XII-37 | $C_2H_5$ | $C_2H_5$ | H | $CH_3$ | H | $C_2H_5$ | Cl | $n_D^{20}$=1,5273 |
| XII-38 | $CH_3$ | $CH_3$ | H | $CH_3$ | $CH_3$ | $C_2H_5$ | Cl | $n_D^{20}$=1,5296 |
| XII-39 | $CH_3$ | $C_2H_5$ | H | $CH_3$ | $CH_3$ | $C_2H_5$ | Cl | $n_D^{20}$=1,5288 |
| XII-40 | $CH_3$ | $CH_3$ | H | $C_2H_5$ | H | $CH_3$ | Cl | $n_D^{20}$=1,5372 |
| XII-41 | $CH_3$ | $CH_3$ | H | H | $CH_3$ | $C_4H_9$ | Cl | $n_D^{20}$=1,5299 |

**Ansprüche**

1. Anilinomethyloxim-ether der Formel

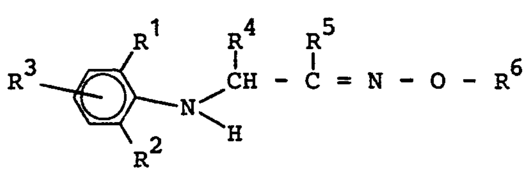

(I)

in welcher

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl oder gegebenenfalls durch Halogen, Alkyl mit 1 bis 4 Kohlenstoffatomen,

Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen steht,

$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl, gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen oder gegebenenfalls im Arylteil durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aralkyl mit 6 bis 10 Kohlenstoffatomen im Arylteil und 1 bis 2 Kohlenstoffatomen im Alkylteil steht und außerdem.

$R^5$ und $R^6$ gemeinsam für eine Alkylen-Gruppe stehen.

2. Verfahren zur Herstellung von Anilinomethyloxim-ethern der Formel

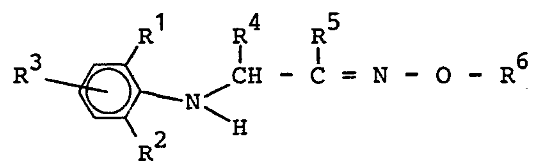

(I)

in welcher

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl oder gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen steht,

$R^6$ für Wasserstoff, Alkyl, Alkenyl, Alkinyl, Alkoxyalkyl, Alkylthioalkyl, Dialkylaminoalkyl, Halogenalkyl, Alkoxycarbonylalkyl, gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen oder gegebenenfalls im Arylteil durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aralkyl mit 6 bis 10 Kohlenstoffatomen im Arylteil und 1 bis 2 Kohlenstoffatomen im Alkylteil steht und außerdem

$R^5$ und $R^6$ gemeinsam für eine Alkylen-Gruppe stehen,

dadurch gekennzeichnet, daß man

a) zur Herstellung von Verbindungen der Formel (I) Aniline der Formel

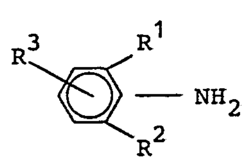

(II)

in welcher

$R^1$, $R^2$ und $R^3$ die oben angegebene Bedeutung haben,

α) mit substituierten Oxim-ethern der Formel

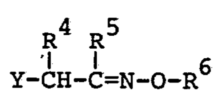

(III)

in welcher

$R^4$, $R^5$ und $R^6$ die oben angegebene Bedeutung haben,

und

Y für Halogen, den Mesylat- oder Tosylat-Rest steht, in Gegenwart eines Säurebinders und

gegebenenfalls in Gegenwart eines Verdünnungsmittels umsetzt, oder

β) mit Propargylhalogeniden der Formel

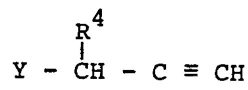

(IV)

in welcher

$R^4$ und Y die oben angegebene Bedeutung haben, in Gegenwart eines Säurebinders und gegebenenfalls in Gegenwart eines Verdünnungsmittels umsetzt, die entstehenden Propargyl-aniline der Formel

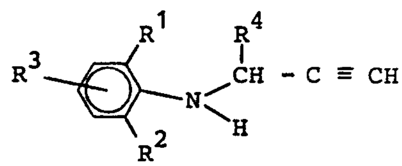

(V)

in welcher

$R^1$, $R^2$, $R^3$ und $R^4$ die oben angegebene Bedeutung haben, in üblicher Weise hydratisiert und die entstehenden Anilin-Derivate der Formel

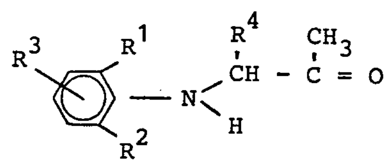

(VI)

in welcher

$R^1$, $R^2$, $R^3$ und $R^4$ die oben angegebene Bedeutung haben, mit Salzen von Hydroxylamin-(Derivaten) der Formel

$$H_2N—O—R^6 \qquad (VII)$$

in welcher

$R^6$ die oben angegebene Bedeutung hat, in Gegenwart eines Verdünnungsmittels und in Gegenwart eines Säurebindemittels umsetzt, oder

γ) mit Keto-Derivaten der Formel

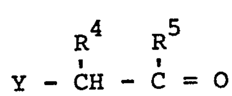

(VIII)

in welcher

$R^4$, $R^5$ und Y die oben angegebene Bedeutung haben, in Gegenwart eines Säurebinders und gegebenenfalls in Gegenwart eines Verdünnungsmittels umsetzt, und die entstehenden substituierten Anilin-Derivate der Formel

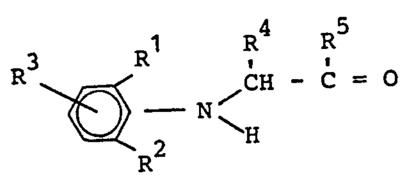

(IX)

in welcher

$R^1$, $R^2$, $R^3$, $R^4$ und $R^5$ die oben angegebene Bedeutung haben, mit Salzen von Hydroxylamin-(Derivaten) der Formel

$H_2N{-}O{-}R^6$ (VII)

in welcher

$R^6$ die oben angegebene Bedeutung hat, in Gegenwart eines Verdünnungsmittels und in Gegenwart eines Säurebindemittels umsetzt, oder

b) zur Herstellung derjenigen Verbindungen der Formel (I), in denen $R^6$ nicht für Wasserstoff steht, Anilin-Derivate der Formel

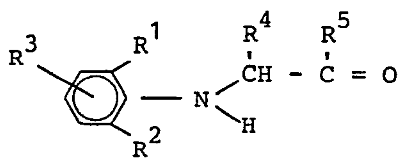

(IX)

in welcher

$R^1$, $R^2$, $R^3$, $R^4$ und $R^5$ die oben angegebene Bedeutung haben, mit Salzen des Hydroxylamins in Gegenwart eines Verdünnungsmittels und in Gegenwart eines Säurebindemittels umsetzt und die dabei entstehenden Oxime der Formel

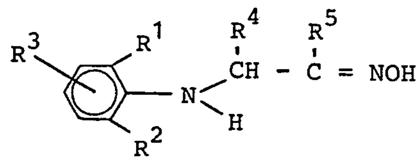

(X)

in welcher

$R^1$, $R^2$, $R^3$, $R^4$ und $R^5$ die oben angegebene Bedeutung haben, in Form ihrer Alkali-Salze mit Dimethylsulfat oder mit Halogeniden der Formel

$X{-}R^7$ (XI)

in welcher

$R^7$ für Alkyl, Alkenyl, Alkinyl, Alkoxyalkyl, Alkylthioalkyl, Dialkylaminoalkyl, Halogenalkyl, Alkoxycarbonylalkyl, gegebenenfalls durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aryl mit 6 bis 10 Kohlenstoffatomen oder gegebenenfalls im Arylteil durch Halogen, Alkyl mit 1 bis 4 Kohlenstoffatomen, Halogenalkyl mit 1 oder 2 Kohlenstoffatomen und 2 bis 5 Halogenatomen, Alkoxy mit 1 oder 2 Kohlenstoffatomen, Alkylthio mit 1 oder 2 Kohlenstoffatomen, Cyano und/oder Nitro substituiertes Aralkyl mit 6 bis 10 Kohlenstoffatomen im Arylteil und 1 bis 2 Kohlenstoffatomen im Alkylteil steht und

X für Chlor oder Brom steht, in Gegenwart eines organischen Verdünnungsmittels oder in Gegenwart eines organisch-anorganischen Zweiphasensystems in Gegenwart eines Phasentransferkatalysators umsetzt, wobei die Alkalisalze der Oxime der Formel (X) in situ erzeugt werden.

**Claims**

1. Anilinomethyl oxime ethers of the formula

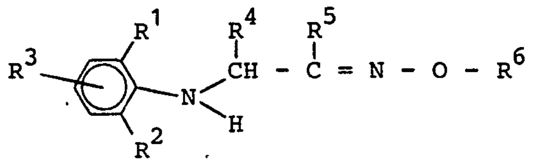

(I)

in which

$R^1$ represents hydrogen, alkyl or alkoxy,

$R^2$ represents hydrogen, alkyl, alkoxy or halogen,

$R^3$ represents hydrogen, alkyl, alkoxy or halogen,

$R^4$ represents hydrogen or alkyl,

$R^5$ represents hydrogen, alkyl or aryl with 6 to 10 carbon atoms which is optionally substituted by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro,

$R^6$ represents hydrogen, alkyl, alkenyl, alkinyl, alkoxyalkyl, alkylthioalkyl, dialkylaminoalkyl, halogenoalkyl, alkoxycarbonylalkyl, aryl with 6 to 10 carbon atoms which is optionally substituted by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro, or aralkyl which has 6 to 10 carbon atoms in the aryl part and 1 to 2 carbon atoms in the alkyl part and is optionally substituted in the aryl part by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro and in addition

$R^5$ and $R^6$ together represent an alkylene group.

2. Process for the preparation of anilinomethyl oxime ethers of the formula

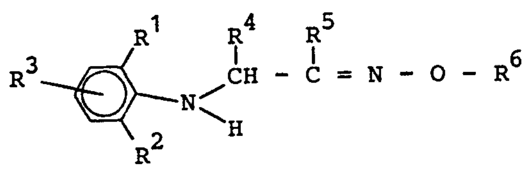

(I)

in which

$R^1$ represents hydrogen, alkyl or alkoxy,

$R^2$ represents hydrogen, alkyl, alkoxy or halogen,

$R^3$ represents hydrogen, alkyl, alkoxy or halogen,

$R^4$ represents hydrogen or alkyl,

$R^5$ represents hydrogen, alkyl or aryl with 6 to 10 carbon atoms which is optionally substituted by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro,

$R^6$ represents hydrogen, alkyl, alkenyl, alkinyl, alkoxyalkyl, alkylthioalkyl, dialkylaminoalkyl, halogenoalkyl, alkoxycarbonylalkyl, aryl with 6 to 10 carbon atoms which is optionally substituted by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro, or aralkyl which has 6 to 10 carbon atoms in the aryl part and 1 to 2 carbon atoms in the alkyl part and is optionally substituted in the aryl part by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro and in addition

$R^5$ and $R^6$ together represent an alkylene group,

characterised in that

a) to prepare compounds of the formula (I) anilines of the formula

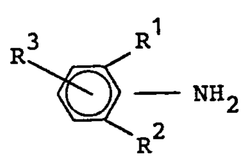

(II)

in which

$R^1$, $R^2$ and $R^3$ have the meaning indicated above,

α) are reacted with substituted oxime ethers of the formula

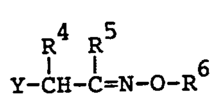

(III)

in which

$R^4$, $R^5$ and $R^6$ have the meaning indicated above and

Y represents halogen, or the mesylate or tosylate radical, in the presence of an acid binder and if appropriate in the presence of a diluent, or

β) are reacted with propargyl halides of the formula

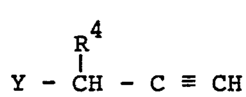

(IV)

in which

$R^4$ and Y have the meaning indicated above, in the presence of an acid binder and if appropriate in the presence of a diluent, the resulting proparnyl anilines of the formula

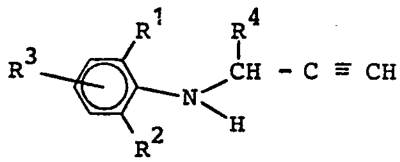

(V)

in which

$R^1$, $R^2$, $R^3$ and $R^4$ have the meaning indicated above, are hydrated in the customary manner and the resulting aniline derivatives of the formula

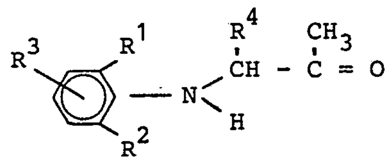

(VI)

in which

$R^1$, $R^2$, $R^3$ and $R^4$ have the meaning indicated above, are reacted with salts of hydroxylamine (derivatives) of the formula

$$H_2N—O—R^6 \qquad (VII)$$

in which

$R^6$ has the meaning indicated, in the presence of a diluent and in the presence of an acid-binding agent, or

γ) are reacted with keto derivatives of the formula

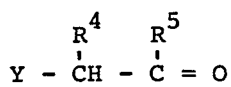

(VIII)

in which

$R^4$, $R^5$ and Y have the meaning indicated above, in the presence of an acid binder and if appropriate in the presence of a diluent, and the resulting substituted aniline derivatives of the formula

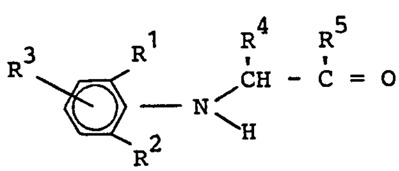

(IX)

in which

$R^1$, $R^2$, $R^3$, $R^4$ and $R^5$ have the meaning indicated above, are reacted with salts of hydroxylamine (derivatives) of the formula

$$H_2N—O—R^6 \qquad (VII)$$

in which

$R^6$ has the meaning indicated above, in the presence of a diluent and in the presence of an acid-binding agent, or

b) to prepare those compounds of the formula (I) in which $R^6$ does not represent hydrogen, aniline derivatives of the formula

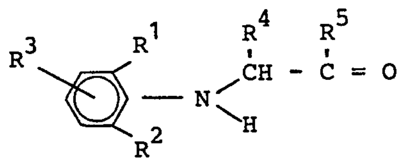

(IX)

in which

$R^1$, $R^2$, $R^3$, $R^4$ and $R^5$ have the meaning indicated above, are reacted with salts of hydroxylamine in the presence of a diluent and in the presence of an acid-binding agent and the resulting oximes of the formula

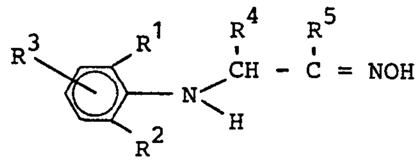

(X)

in which

$R^1$, $R^2$, $R^3$, $R^4$ and $R^5$ have the meaning indicated above, are reacted in the form of their alkali metal salts with dimethyl sulphate or with halides of the formula

$$X—R^7 \qquad (XI)$$

in which

$R^7$ represents alkyl, alkenyl, alkinyl, alkoxyalkyl, alkylthioalkyl, dialkylaminoalkyl, halogenoalkyl, alkoxycarbonylalkyl, aryl with 6 to 10 carbon atoms which is optionally substituted by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro or aralkyl which has 6 to 10 carbon atoms in the aryl part and 1 to 2 carbon atoms in the alkyl part and is optionally substituted in the aryl part by halogen, alkyl with 1 to 4 carbon atoms, halogenoalkyl with 1 or 2 carbon atoms and 2 to 5 halogen atoms, alkoxy with 1 or 2 carbon atoms, alkylthio with 1 or 2 carbon atoms, cyano and/or nitro and

X represents chlorine or bromine, in the presence of an organic diluent or in the presence of an organic-inorganic two-phase system in the presence of a phase transfer catalyst, the alkali metal salts of the oximes of the formula (X) being produced in situ.

**Revendications**

1. Ethers d'anilinométhyloximes de formule

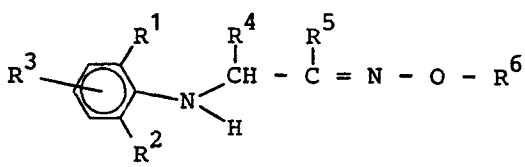

(I)

dans laquelle

$R^1$ représente l'hydrogène ou un groupe alkyle ou alcoxy,

$R^2$ représente l'hydrogène ou un groupe alkyle ou alcoxy ou un halogène,

$R^3$ représente l'hydrogène ou un groupe alkyle ou alcoxy ou un halogène,

$R^5$ représente l'hydrogène ou un groupe alkyle, ou un groupe aryle en $C_6$-$C_{10}$ éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro,

$R^6$ représente l'hydrogène ou un groupe alkyle, alcényle, alcynyle, alcoxyalkyle, alkylthioalkyle, dialkylaminoalkyle, halogénoalkyle, alcoxycarbonylalkyle, ou un groupe aryle en $C_6$-$C_{10}$ éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro ou un groupe arylalkyle en $C_6$-$C_{10}$ dans la portion aryle et en $C_1$-$C_2$ dans la portion alkyle éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro et, en outre,

$R^5$ et $R^6$ représentent ensemble un groupe alkylène.

2. Procédé pour la fabrication d'éthers d'anilino-méthyloximes de formule

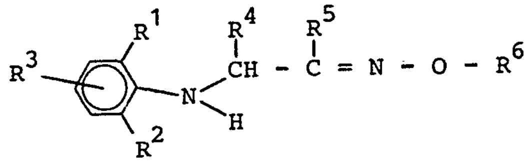

(I)

dans laquelle

$R^1$ représente l'hydrogène ou un groupe alkyle ou alcoxy,

$R^2$ représente l'hydrogène ou un groupe alkyle ou alcoxy ou un halogène,

$R^3$ représente l'hydrogène ou un groupe alkyle ou alcoxy ou un halogène,

$R^4$ représente l'hydrogène ou un groupe alkyle,

$R^5$ représente l'hydrogène ou un groupe alkyle ou un groupe aryle en $C_6$-$C_{10}$ éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro,

$R^6$ représente l'hydrogène ou un groupe alkyle, alcényle, alcynyle, alcoxyalkyle, alkylthioalkyle, dialkylaminoalkyle, halogénoalkyle, alcoxycarbonylalkyle, ou un groupe aryle en $C_6$-$C_{10}$ éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio ou $C_1$ ou $C_2$, cyano et/ou nitro ou un groupe arylalkyle en $C_6$-$C_{10}$ dans la portion aryle et en $C_1$-$C_2$ dans la portion alkyle éventuellement substitué dans la portion aryle par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro et, en outre,

$R^5$ et $R^6$ représentent ensemble un groupe alkylène,

caractérisé en ce que

a) pour la fabrication de composés de formule (I), on fait réagir des anilines de formule

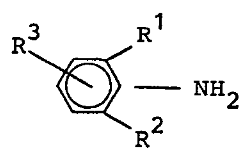

(II)

dans laquelle

$R^1$, $R^2$ et $R^3$ ont la signification indiquée ci-dessus,

α) avec des éthers d'oximes substitués de formule

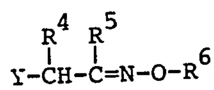

(III)

dans laquelle

$R^4$, $R^5$ et $R^6$ ont la signification indiquée ci-dessus et Y représente un halogène, le reste mésylate ou le reste tosylate, en présence d'un accepteur d'acide et éventuellement en présence d'un diluant, ou bien

β) avec des halogénures de propargyles de formule

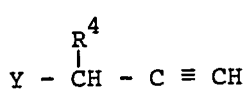

(IV)

dans laquelle

$R^4$ et Y ont la signification indiquée ci-dessus, en présence d'un accepteur d'acide et éventuellement en présence d'un diluant, on hydrate de manière habituelle les propargylanilines formées de formule

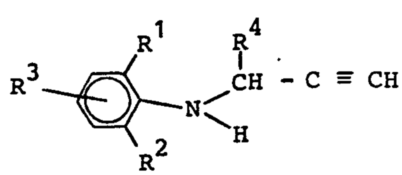

(V)

dans laquelle

$R^1$, $R^2$, $R^3$ et $R^4$ ont la signification indiquée ci-dessus, et on fait réagir les dérivés d'aniline formés de formule

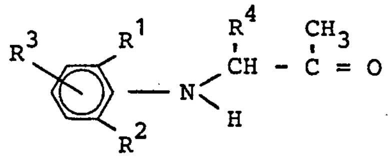

(VI)

dans laquelle

$R^1$, $R^2$, $R^3$ et $R^4$ ont la signification indiquée ci-dessus, avec des sels (de dérivés) d'hydroxylamine de formule

$$H_2N—O—R^6 \qquad (VII)$$

dans laquelle

$R^6$ a la signification indiquée ci-dessus, en présence d'un diluant et éventuellement en présence d'un accepteur d'acide, ou bien

γ) avec des dérivés céto de formule

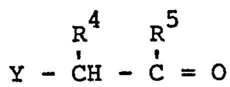

(VIII)

dans laquelle

$R^4$, $R^5$ et Y ont la signification indiquée ci-dessus, en présence d'un accepteur d'acide et éventuellement en présence d'un diluant, et on fait réagir les dérivés d'aniline substitués formés de formule

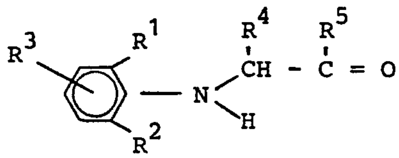

(IX)

dans laquelle

$R^1$, $R^2$, $R^3$ et $R^4$ et $R^5$ ont la signification indiquée ci-dessus, avec des sels (de dérivés) d'hydroxylamine de formule

$$H_2N—O—R^6 \qquad (VII)$$

dans laquelle

$R^6$ a la signification indiquée ci-dessus, en présence d'un agent diluant et en présence d'un accepteur d'acide, ou bien

b) pour la fabrication des composés de formule (I) dans lesquels $R^6$ ne représente pas l'hydrogène, on fait réagir des dérivés d'aniline de formule

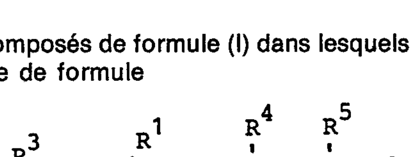

(IX)

dans laquelle

$R^1$, $R^2$, $R^3$, $R^4$ et $R^5$ ont la signification indiquée ci-dessus, avec des sels de l'hydroxylamine, en présence d'un agent diluant et en présence d'un accepteur d'acide et on fait réagir les oximes formées de formule

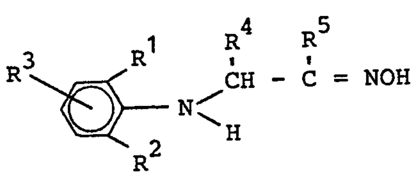

(X)

dans laquelle

$R^1$, $R^2$, $R^3$, $R^4$ et $R^5$ ont la signification indiquée ci-dessus, sous forme de leurs sels alcalins, avec le sulfate de diméthyle ou avec des halogénures de formule

$$X—R^7 \qquad (XI)$$

dans laquelle

$R^7$ représente un groupe alkyle, alcényle, alcynyle, alcoxyalkyle, alkylthioalkyle, dialkylaminoalkyle, halogénoalkyle, alcoxycarbonylalkyle, aryle en $C_6$-$C_{10}$ éventuellement substitué par halogène, alkyle en $C_1$-$C_4$, halogénoalkyle en $C_1$ ou $C_2$ et ayant 2 à 5 atomes d'halogènes, alcoxy en $C_1$ ou $C_2$, alkylthio en $C_1$ ou $C_2$, cyano et/ou nitro, ou un groupe arylalkyle en $C_6$-$C_{10}$ dans la portion aryle et en $C_1$-$C_2$ dans la portion alkyle éventuellement substitué dans la portion aryle par halogène, alkyle en $C_1$-$C_4$, halogénoalkyle en $C_1$ ou $C_2$ et ayant 2 à 5 atomes d'halogène, alcoxy en $C_1$ ou $C_2$, alkylthio en $C_1$ ou $C_2$, cyano et/ou nitro, et

X représente le chlore ou le brome, en présence d'un diluant organique ou en présence d'un système à deux phases organique-inorganique, en présence d'un catalyseur de transfert de phase, les sels alcalins des oximes de formule (X) étant produits in situ.